# EUROPEAN PATENT APPLICATION

(11) **EP 1 389 442 A1**
(43) Date of publication of application: **18.02.2004**
(21) Application number: 03018481.6
(22) Date of filing: 14.08.2003
(51) Int. Cl.: A61B 5/04

(54) **Probe suitable for use in neural tissue**

(30) Priority: 16.08.2002 SE 0202448
(71) Applicant: Hofmann, Ulrich, 23562 Lübeck (DE); Norlin, Peter, 75335 Uppsala (SE); Kindlundh, Maria, 19162 Sollentuna (SE)
(72) Inventor: Hofmann, Ulrich, 23562 Lübeck (DE); Norlin, Peter, 75335 Uppsala (SE); Kindlundh, Maria, 19162 Sollentuna (SE)
(74) Representative: Biehl, Christian, Dipl.-Phys.

(57) **Abstract**

A probe is disclosed, the probe comprising at least one shaft and at least one contact pad. Each shaft is suitable for insertion into neural tissue and has a number of electrodes. Each contact pad is in electrical contact with a set of the electrodes and at least one electrode is at least partially covered by an electrically insulating layer. Furthermore a manufacturing process for such a probe is disclosed.

## Description

### Field of the invention

This invention refers to a neural probe suitable for insertion into neural tissue and a method for manufacturing such a probe.

### Technological background

Neural probes are used for neuroscientific purposes. The idea is to insert a number of electrodes into the neural tissue to be studied. The electrodes can be used for recording neural signals as well as for emitting signals that stimulate the nerve cells. To facilitate relevant readings, it is of outmost importance for the electrodes to be placed accurately in the tissue. This can, for example, be achieved using a fork-shaped probe having a base plate and a number of shafts. In such case, the electrodes are provided in pre-defined positions on the shafts, and a conductor strip connects each electrode to a connection pad located on the base plate. By inserting the shafts into the tissue, the electrodes are put into position. By connecting the probe to a signal amplifier and processing the amplified signals in a computer means, the neural signals can be studied and analysed.

Micro System Technology is well suited for batch-fabrication of fork-like probes with multi-electrodes, intended for recording or stimulation in neural tissue. However, a fundamental problem is that the desired spatial distributions of the electrodes usually are different for different neuroscientific experiments. This presents a major problem, since the manufacturing process cannot easily be adjusted for different designs. Generally adjustments would imply redesigning the entire lithographic mask-set of the probes. In scenarios with commercially offered neural probes, custom made designs to individual researchers will be close to impossible due to the associated costs and lead times. Thus, the only economically defendable approach is to manufacture a small number of standard probe designs. These designs then have to be used, even if the resulting electrode distribution is far from optimal for the experiment at hand.

Furthermore, it is important for the shafts to be as thin as possible, not to damage the tissue when inserted. Therefore, the number of electrode sites is limited by the number of conductor strips that can possibly be placed on a shaft having an acceptable width.

The German utility model application DE 20115311 presents an approach based on a so-called pre-product. The pre-product, having the customary fork-shape, comprises a number of connection pads. The pads are located on a base plate of the probe and each pad is related to a plurality of electrodes. The connection pads can selectively be connected to any one of its related electrodes. Thus, a multiple of electrode distributions can be provided using the same type of pre-product, whilst the shafts only need to accommodate as many conductors as the number of electrodes on each shaft comprised in the electrode distributions, i.e. the number of contact pads related to electrodes on each shaft.

There are two slightly different principles disclosed in DE 20115311. According to the first principle, each connection pad is connected to a conductor strip on one of the shafts, and the electrodes related to the connection pad are connected to the conductor strip in default. By disconnecting every electrode not needed, a desired electrode distribution is generated. To disconnect an electrode, it is disconnected from the conductor strip using laser cutting or micromechanic cutting.

According to the second principle, each connection pad is connected to a conductor strip on a shaft, similar to the first principle, but the electrodes related to the connection pad is disconnected from the conductor strip in default. Instead of diconnecting the electrodes not wanted in the desired electrode distribution, the electrodes wanted is connected to the conductor strip. This is done either by electron beam deposition or by attaching some kind of connecting element.

Thus, whichever principle used, the number of conductor strips can be kept as low as the number of electrodes in the electrode distribution. However, even though only one conductor is needed for each electrode of the electrode distribution, extra space is needed on each shaft to facilitate the connection or disconnection of the electrodes. This results in the shafts having to be wider and thus causing unnecessary damage to the tissue in which the probe is to be inserted. Locating the connection/disconnection area on the base plate would necessitate as many conductor strips as the number of electrodes on each shaft, and thus completely eradicate the advantage of the idea.

One method of fabricating neural probes such as the ones described in DE 20115311 is disclosed in "A 32-site neural recording probe fabricated by DRIE of SOI substrates", Journal of Micromechanics and Microengineering, 12 2002, pp 414-419, P. Norlin et al. The described method involves several steps utilizing Reactive Ion Etching (RIE) through resist masks, which have been lithographically patterned by means of exposure through glass/chrome photo-masks. Such photo-masks are complicated to manufacture, therefore customized etchings are expensive and involve extensive lead times.

Furthermore, the techniques described in DE 20115311 for establishing or breaking the contacts is quite complicated. Either a micromechanical cutting process, a laser cutting device or an electron beam deposition device is needed. Alternatively, the contacts might be provided by wire elements, which is extremely time consuming and results in even larger connection areas.

In summary, even though the pre-product disclosed in DE 20115311 presents a flexible solution to the problem related to different electrode distributions, the selective connection and disconnection of electrodes is complicated and still results in shafts being unnecessary wide. Thus, there is a need for refined neural probes, which provides smaller shaft dimensions and which can be programmed using more commonly available equipment.

### Summary of the invention

One object of the invention is to provide probes suitable for use in neural tissue, which have compact dimensions and can be customized on-demand to provide a large number of various electrode distributions. This is achieved by a neural probe as claimed in claim 1 and by the manufacturing process as claimed in claim 1M. The appended sub-claims specify various advantageous embodiments of the invention.

It is realized, that the dimensions of the probe shafts can be reduced, as compared to the probes described in DE 20115311, if no connection arrangements are needed between the contact pads and the related electrodes. Thus, according to the invention, the neural probe comprises a number of contact pads, each contact pad having electrical contact with a number of electrodes at predetermined positions, similar to the first principle described in DE 20115311. However, instead of etching windows on every electrode, using a fixed pattern resist mask, only the electrodes needed to be active for the experiment at hand is opened. The other electrodes are left insulated. Thus, a desired electrode distribution is generated by removing the insulation layer from some predetermined set of electrodes, the predetermined set defining the desired distribution, and leaving the insulation on the rest of the electrodes. By removing the insulation from an electrode, the electrode is activated. In other words, instead of connecting or disconnecting an electrode by interfering with its electrical connection, it is deactivated by an insulating layer between the electrode and its surroundings and activated by the removal of the insulating layer. Thereby, the circuit strips and the electrode connections can be given a more compact design, enabling less wide shafts. The positions of the activated electrodes determine the desired electrode site distribution whilst the deactivated electrodes are passive and not used. There are many different ways of removing the insulation from an electrode.

Selective opening of windows in a passivation layer, i.e. insulation layer, has the further advantage of facilitating variable electrode areas. This is easily achieved by varying the size of the windows in the insulating layers. This is useful for many applications. In particular, a smaller electrode area facilitates more spatially selective readings. However, a smaller area also results in higher impedance, which in turn results in higher thermal noise. Thus, in general when designing neural probes, there is a trade-off between spatial selectivity and low thermal noise of the electrodes. The possibility to vary the window area on-demand, enables this trade off between the signal-to-noise ratio and the selectivity to be adjusted for every individual experiment.

Of course, there might be other reasons for varying the electrode area. Whichever the reason is, the invention provides a feasible way of achieving this.

However, other programming techniques could be considered as well, e.g. programmable fuses, laser cutting or ultra-sonic cutting of conductors or laser destruction of link insulators.

According to one aspect of the invention, a method of manufacturing a neural probe having at least one shaft is provided. The method involves providing at least one set of electrodes on the shaft. The electrodes are located at respective electrode sites and the electrodes of each set are electrically interconnected. One alternative is to provide only one set of electrodes. However, the larger the number of sets, the larger the number of possible electrode distributions. Basically, since each set needs a separate conductor, the number of electrode sets on a shaft is restricted by the number of conductors possible to accommodate on the shaft given its dimensions.

The method further involves applying an electrically insulating layer onto every electrode. Either one single layer can be applied to all the electrodes, or individual layers can be applied to each one of the electrodes. The layer is such that when the probe is in use, the electrode has no significant electrical contact with its surroundings. In particular, when inserted in neural tissue, an insulated electrode is inoperative to register or emit any electrical nerve signal. After having applied the insulating layer/s, the layer/s is/are at least partially removed from every electrode comprised in desired electrode distribution. By removing the layer from an electrode, the electrode will, when in use, have electrical contact with its surroundings. Thus, the electrode will be operative to register as well as to emit electrical nerve signals. The desired electrode distribution can be chosen as any combination of the electrodes. However, since electrodes of the same set are interconnected, the distribution can only comprise as many electrically separated electrodes as the number of electrode sets. Thus, the spatial distribution of the electrodes as well as their mutual electrical interconnections restricts the number of available electrode distributions to choose between.

One preferred embodiment for removing the insulation from an electrode is by means of a direct write lithography (DWL) equipment, during the fabrication of the probes. According to this embodiment, the fabrication of the probes is carried out much the same as is described in the above cited "A 32-site neural recording probe fabricated by DRIE of SOI substrates". However, instead of defining the resist mask pattern for the etch of the final silicon nitride layer with fixed glass/chrome photomasks, DWL equipment is used for the resist patterning. By using a DWL technique, the need for one photo-mask for each pattern to be etched is eliminated. The DWL equipment can easily be computer programmed to open electrode windows on only a predetermined set of electrodes, leaving the other electrodes insulated.

The general drawback of DWL is its low throughput. This application, however, turns out to be ideally suited for DWL, as only one mask layer needs to be written and the complexity of the pattern is limited. DWL equipment is expected to be easier to handle and more readily available as compared to the equipment needed to program the pre-products described in DE 20115311.

Other direct write methods might be used as well, having similar advantages as compared to a glass/chrome photomask.

A detailed description of DWL techniques in general, and optical DWL in particular can be found in "Heidelberg Instruments DWL 200 / 400 User Guide", Part I-II, Doc. no. DWL-HI-011, © Heidelberg Instruments Mikrotechnic, 2001.

Of course, instead of using DWL equipment, is possible to define a fixed glass/chrome photomask for each desired electrode distribution. Thereby, the need for DWL equipment is eliminated at the cost of more complicated on-demand customizations. This approach might, for example, be interesting for applications where a limited number probe designs having different window areas are requested.

According to another aspect, the invention provides a probe comprising at least one shaft and at least one contact pad. Each shaft is suitable for insertion into neural tissue and has a number of electrodes spatially distributed on it. Each contact pad has electrical contact with a set of the electrodes. The electrical contact can, for example, be provided by conducting strips, leading from the contact pads to their related electrodes. Furthermore, at least one electrode is at least partially covered by an electrically insulating layer. Thus, some electrodes can be fully covered by an insulating layer, whilst other electrodes are only partially covered or not covered at all. An electrode fully covered by an insulating layer will be inoperative when the probe is in use, i.e. not being able to emit or register nerve signals.

It is to be understood, that the insulating layer can be removed in different ways. One alternative is to remove the layer completely from a part of the electrode, or from the complete electrode. Another alternative is to alter the thickness of the layer. An electrode covered by a thin enough layer will have partial electrical contact with a surrounding tissue, i.e. have very high impedance.

It is also envisaged to alter the activation and deactivation of electrodes of existing probes. This can be done by applying an insulating layer or removing an insulating layer from an electrode. An insulating layer can, for example, by applied by means of a printing process. Of course, this also facilitates varying of the electrode window area.

The invention thus describes a technique to vary the spatial distribution of the electrodes on-demand, in order to meet different experimental requirements, without having to redesign the entire lithographic mask-set of the probes. For a given shaft width and conductor line width, the number of conductors on each shaft is limited. To have different electrode distributions selectively activated using the same conductors is thus a way to make efficient use of available space. The invention further provides the possibility to vary the trade-off between the signal-to-noise ratio and the spatial selectivity.

### Brief description of the drawings

The invention will now be further explained with reference to the attached drawings, which illustrates preferred embodiments of the invention.

Figure 1 schematically illustrates an inventive neural probe, having a base plate and four shafts.

Figure 2 shows a schematic top view of a probe similar to the one shown in Figure 1, as well as a magnified end portion of a shaft of the probe.

Figure 3 diagrammatically illustrates three different electrode site distributions, generated by a shaft having 8 conductor strips and 16 electrodes.

Figure 4 shows a SEM (Scanning Electron Microscope) picture of end portions of the shafts of a probe having eight shafts.

Figure 5 schematically illustrates a portion of a shaft having selectively opened electrode windows.

Figure 7 illustrates the manufacturing process of an inventive neural probe.

Figure 8 illustrates the principles of a DWL equipment.

Figure 9 illustrates the impedance at different frequencies for not contacted shaft as well as for opened and closed electrode window.

Figure 10 illustrates the impedance for different electrode window areas.

Figure 11 illustrates a possible "snap grid" for on-demand probes.

Figure 12 schematically shows two shaft portions, each having different electrode window distributions.

Figure 13 shown schematically illustrates a possible set of five standard different probe designs, as well as their corresponding three different shaft designs.

Figure 14 illustrates the parameter spaced covered by the suggested set of probes illustrated in Figure 13.

### Detailed description of the invention

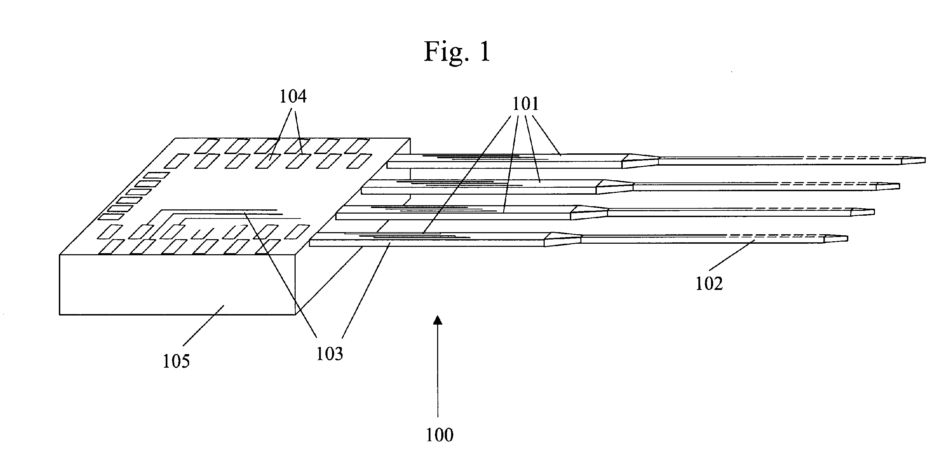

Figure 1 illustrates one embodiment of an inventive neural probe 100, having a base plate 105 and four shafts 101. A number of contact pads 104 are located on the base plate 105, each contact pad 104 being electrically connected to a number of electrodes 102 located on the shafts 101. Preferably, all the electrodes 102 connected to the same contact pad 104 are located on the same shaft 101. The electrical connection is provided by conducting strips 103, leading from each contact pad 104 to its related electrodes 102.

In one preferred embodiment, the shafts 101 are made out of Si structures, the electrodes are made out of Pt or Ir and the conducting strips 103 and contact pads 104 are made out of Au. The base plate 105 can be made out of Si and is preferable thicker than the shafts 101 to allow easy handling of the probes.

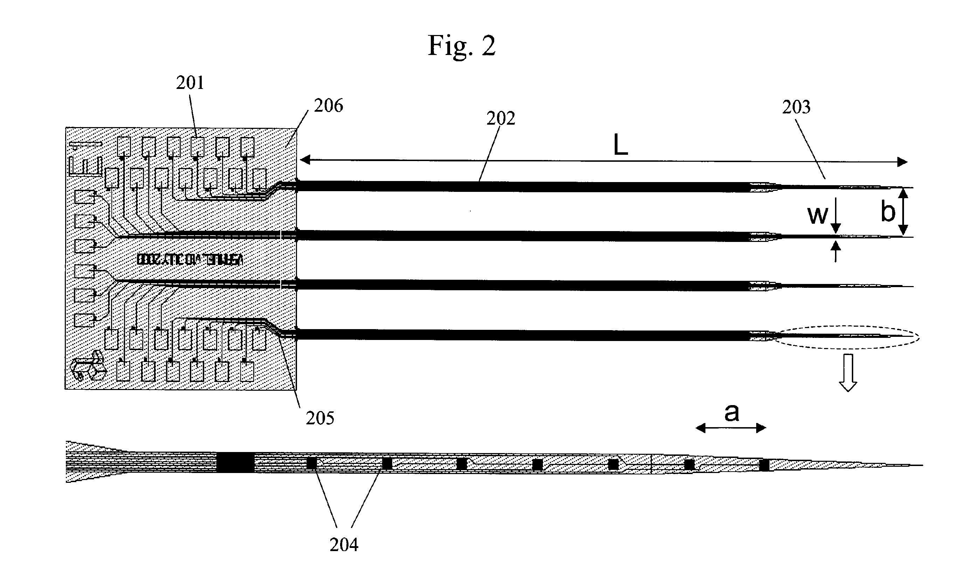

Figure 2 illustrates a top view of a neural probe similar to the one described with reference to Figure 1. The distribution of the conducting pads 201 on the base plate 206 is clearly visible. A close look reveals four groups of conducting pads 201, each group being connected to the electrodes on one of the four shafts 202, by the conducting strips 205. In this embodiment, each shaft has an end portion 203 being thinner than the rest of the shaft, illustrated of the magnification in Figure 2, on which end portion 203 the electrodes are distributed. A thinner end portion 203 reduces the damage caused when the shaft is inserted into neural tissue.

From a user perspective, the main design parameters of a neural probe are (1) the electrode site c/c distance *a*, (2) the shaft c/c distance *b,* and (3) the shaft length *L*. Together the parameters a and *b* define a 2-dimensional array of electrode sites at which individual measurements (or stimulations) can take place. The invention describes a technique to vary the spatial distribution of this array in order to meet different experimental requirements, without having to redesign the entire lithographic mask-set of the probes.

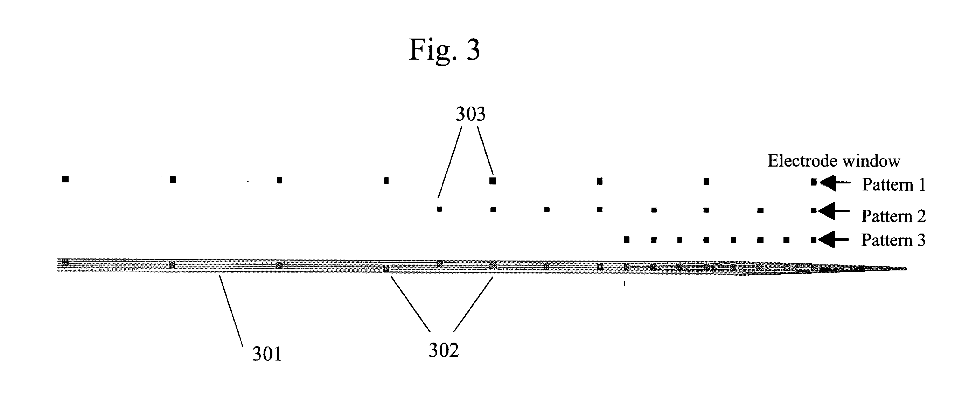

Figure 3 discloses a sample CAD-design of an "electrode window programmable" probe. The probe 301, hawing a multiple of electrodes 302, can be programmed to three different electrode c/c site distances (e.g. 50 µm, 100 µm and 200 µm) by selecting one of the three electrode window patterns (shown as pattern 1, 2 and 3). The squares 303 schematically illustrates the positions of electrodes having opened windows, i.e. being activated.

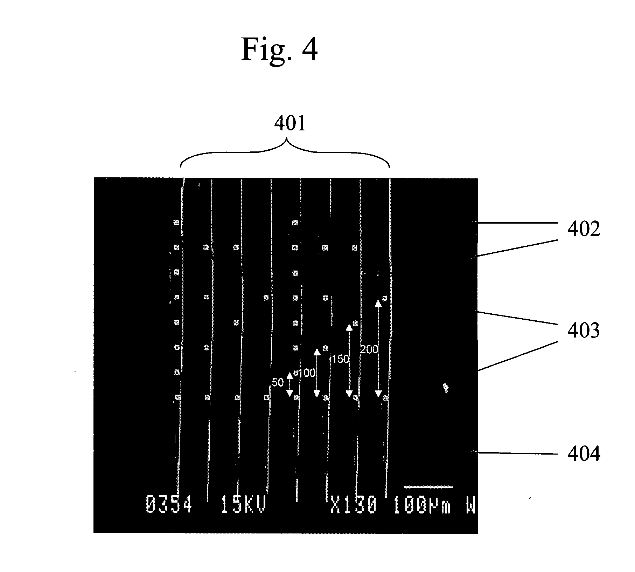

Figure 4 shows a SEM picture of the end portions 404 of a set 401 of eight shafts. The shafts have selectively opened electrode windows (bright squares, 403) with varying electrode distances (e.g. c/c 50 µm, 100 µm and 200 µm) as well as electrodes with closed windows (dark squares, 402). Figure 4 thus shows a SEM picture of an inventive neural probe, on which the electrode distance is varied by selective opening of windows in a top Si₃N₄-layer using DWL and Reactive Ion Etching.

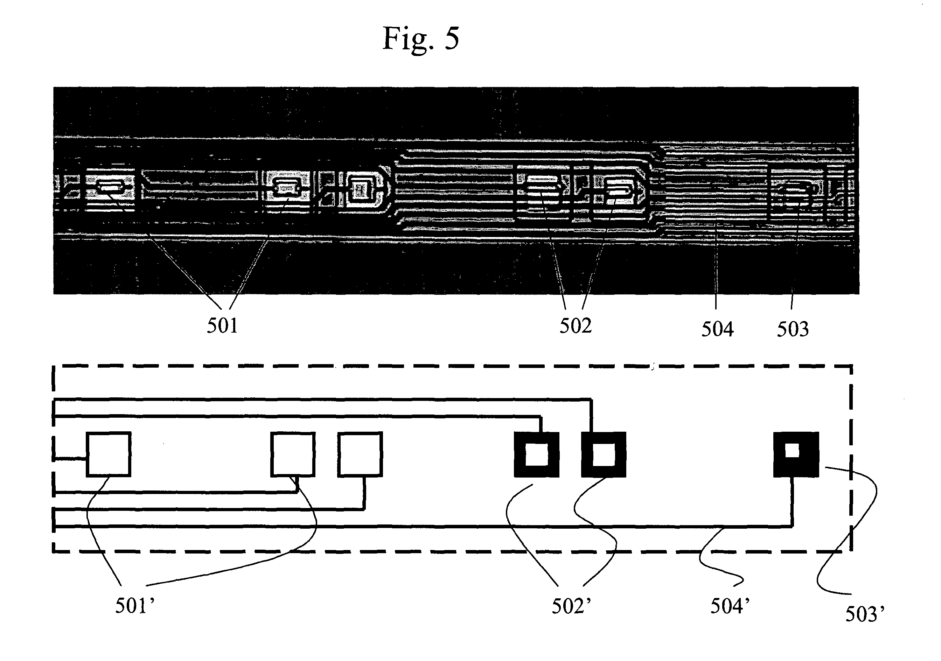

Figure 5 illustrates a portion of a shaft (upper picture) having a number of conducting strips 504 being connected to electrodes 501, 502, 503. The electrodes have differently sized windows opened on them. Electrode 503 has a small window, electrodes 502 has larger windows and electrodes 501 has fully opened windows. For clarity, a schematic drawing (lower picture) illustrating the same portion is disclosed. The schematic drawing illustrates the electrodes having a small window 503', larger windows 502' and fully opened windows 501'. The conducting strips 504' is only disclosed schematically and the black areas illustrate the electrically insulating layers on the electrodes.

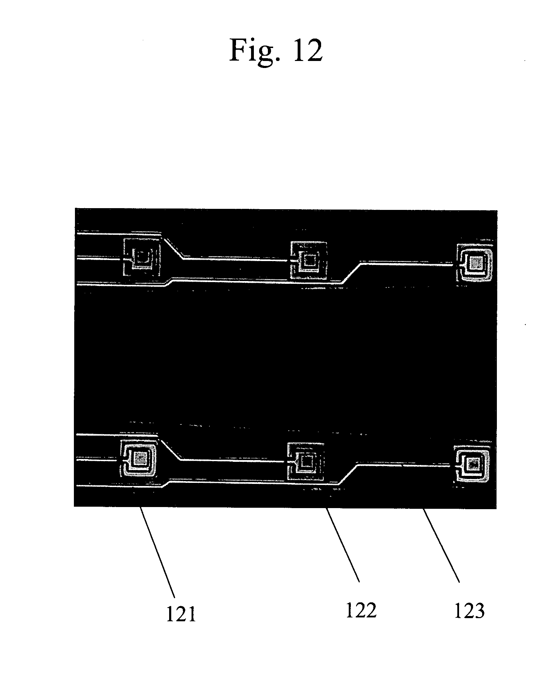

Figure 12 discloses a photomicrogram of selectively opened electrode windows 121 and closed electrode windows 122. Also shown is some clearly separated conducting strips 123.

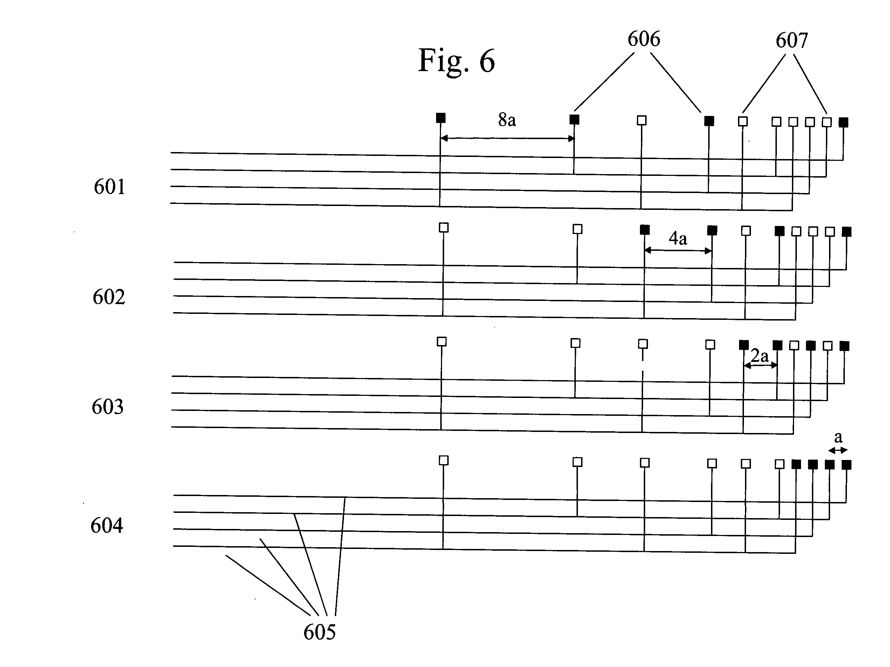

Figure 6 schematically illustrates four different electrode distributions 601, 602, 603, 604 possible to generate on an embodiment of the invention having four conducting strips 605 and 10 electrodes 606, 607. Electrodes having opened windows are illustrated by black squares 606 and electrodes having closed windows are illustrated by white squares 607.

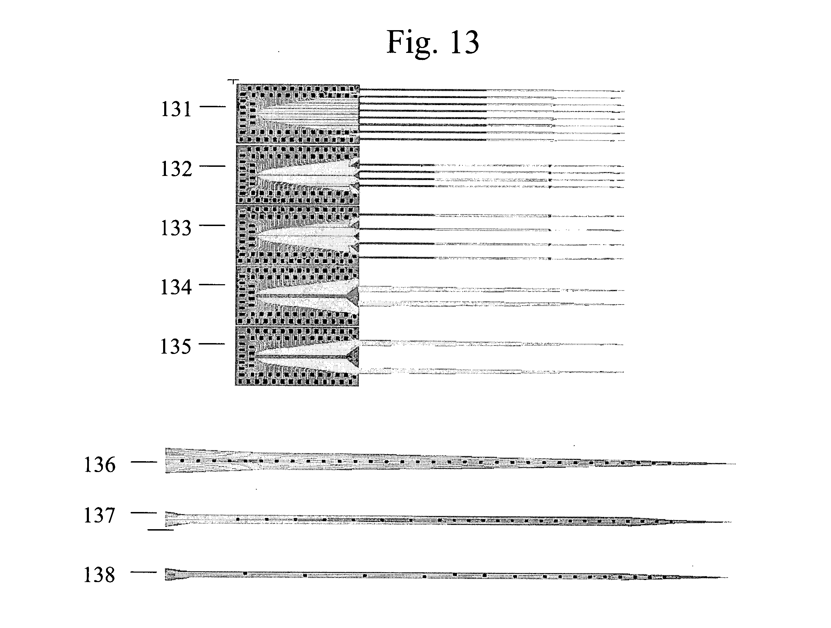

Even if an inventive neural probe provides a large parameter space for the electrode site distribution, it is not possible to provide a probe from which any given pattern can be generated. This is partially due to the fact that there is a discrete set of electrodes, connected to a limited number of conductor strips, but also due to the distances between the shaft being fixed when manufacturing the probe (shown as the measure b in Figure 2). Therefore, it could still be useful to provide a set of different standard designs, each design providing different sets of possible electrode distributions. Figure 13 illustrates a set of five different designs, 131, 132, 133, 134, 135, differing in the number of shafts as well as in the shaft distances. Figure 13 also illustrates three different shaft designs utilized by the probes. Shaft 136 is used for probe 131, shaft 137 is used for probe 132 and probe 133, and shaft 137 is used for probe 134 and probe 135.

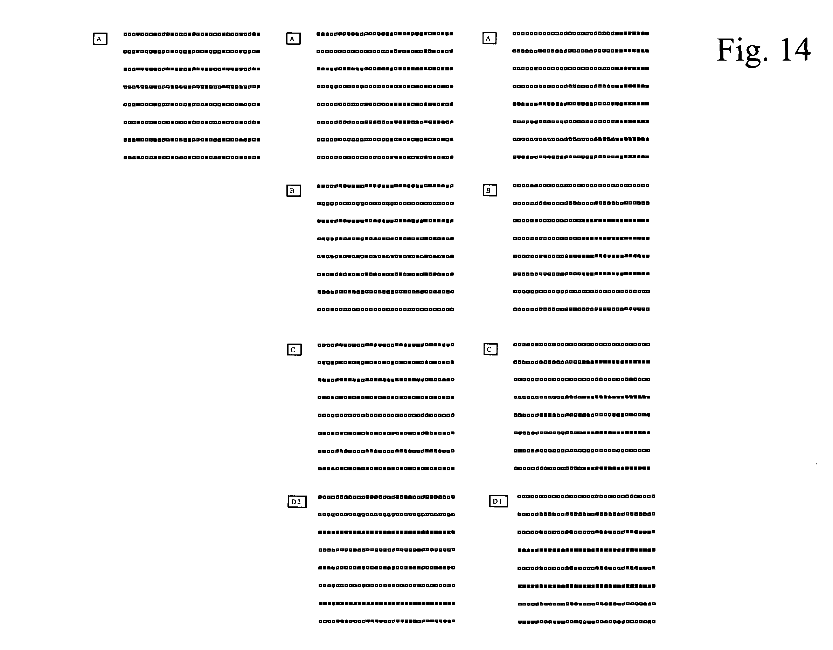

Figure 14 illustrates some electrode distribution possible to generate with the probes shown in Figure 13. The distributions denoted A, B, C, D1, D2 refers to probe 131, 132, 133, 134, and 135, respectively.

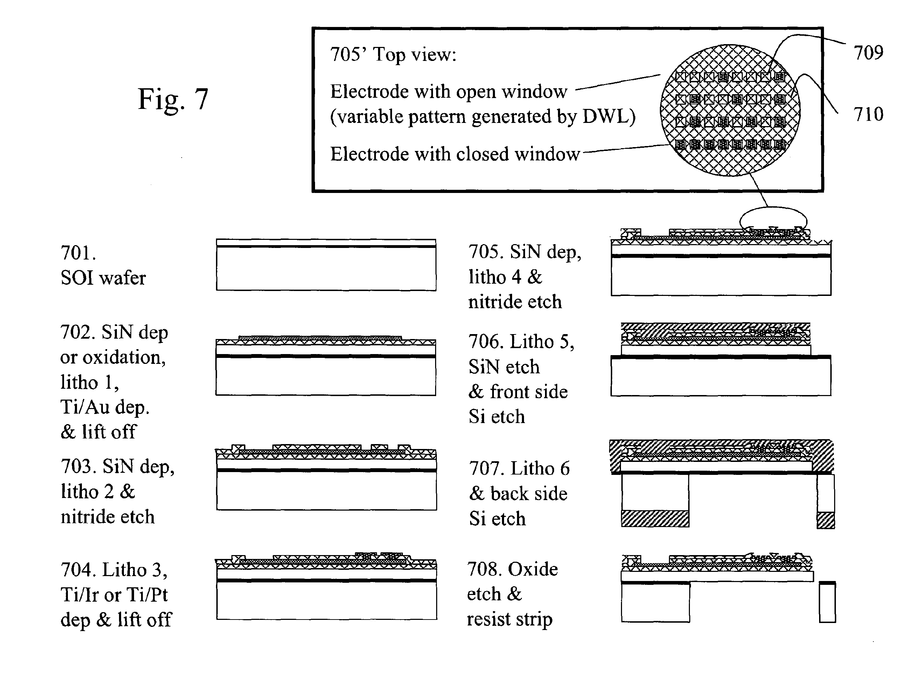

One preferred manufacturing process for the inventive neural probe is illustrated in Figure 7.

According to this embodiment, the probes are manufactured on silicon-on-insulator (SOI) substrates 701 (Shin Etsu, 100 mm Ø, 525 µm Si/1.5 µm SiO₂ / 20 µm Si). To this end, a PECVD (Plasma Enhanced Chemical Vapor Deposition) silicon nitride film (1 µm thick) is deposited 702 as an insulating layer on the substrate. Thereafter Ti/Au (∼500 Å/2500 Å) is e-beam evaporated and patterned with a photo resist lift-off process, to form conductor traces. Preferably five times reduction step-and-repeat projection lithography is used with down to 1 µm linewidths and spacing. Having formed the conductor traces, a second silicon nitride layer (0.5 µm) is deposited 703 as an intermediate dielectric. Thereafter via holes are opened to the Au-layer using Reactive Ion Etching (RIE) through the resist mask, and Ti/Ir (∼300 Å/3500 Å) is e-beam evaporated and patterned 704 with lift off, to form the electrode sites.

A final silicon nitride layer (0.5 µm) is deposited 705 as a protective layer. Thereafter, differently spaced and sized electrode windows are defined by RIE etching of the nitride layer through a patterned resist mask, defined by exposure in a direct write laser pattern generator (Heidelberg Instruments DWL 2.0). The nitride covers the edges of the metal patterns for increased reliability in wet working environments. 705' illustrates a magnified top view of the electrode during the manufacturing process. The electrodes are shown as squares 709, 710, the white squares 709 illustrating windows to be opened and the black squares 710 illustrating windows to be left closed.

Using a resist mask, the remaining nitride layer is first RIE etched 706, after which the top silicon layer is etched 20 µm down to the buried oxide in an inductively coupled plasma deep reactive ion etching equipment (ICP DRIE, Surface Technology Systems). Thereafter, a thick resist is spun 707 on the wafer front side for protection. A double-sided mask alignment (Karl Suss MA6) is used to pattern a thick resist on the wafer back side, which was etched the full 525 µm down to the buried oxide in the ICP DRIE. Finally the shafts are released 708 by first etching the buried oxide in buffered HF, followed by a final resist strip.

In general micromachined neural probes are manufactured starting with a silicon wafer. On the wafer are typically deposited or grown: (a) electrically conducting layers with the purpose of recording or transferring electrical signals, (b) dielectric layers with the purpose of isolating the electrically conducting structures from each other or the environment, or intended as intermediate etch masks. The dielectric or conducting layers are shaped into desired structures using wet or dry etching processes masked with patterned photo-resist layers. Patterning is accomplished with photolithographic processes, typically UV-light exposure of the resist in the desired pattern. In order to create thin and narrow shafts as the mechanical carriers of the signal recording structures, the silicon wafer itself is shaped into the desired structures using wet or dry etching processes. This etching process can typically be controlled by patterned resist, doping of the silicon (etch-stop), buried oxide layers, or a combination of these methods. The manufacturing methods are usually borrowed from, or reminiscent of, those known as integrated circuit (IC) technology. In general, other structural materials than silicon are also conceivable, e.g. metals or polymers.

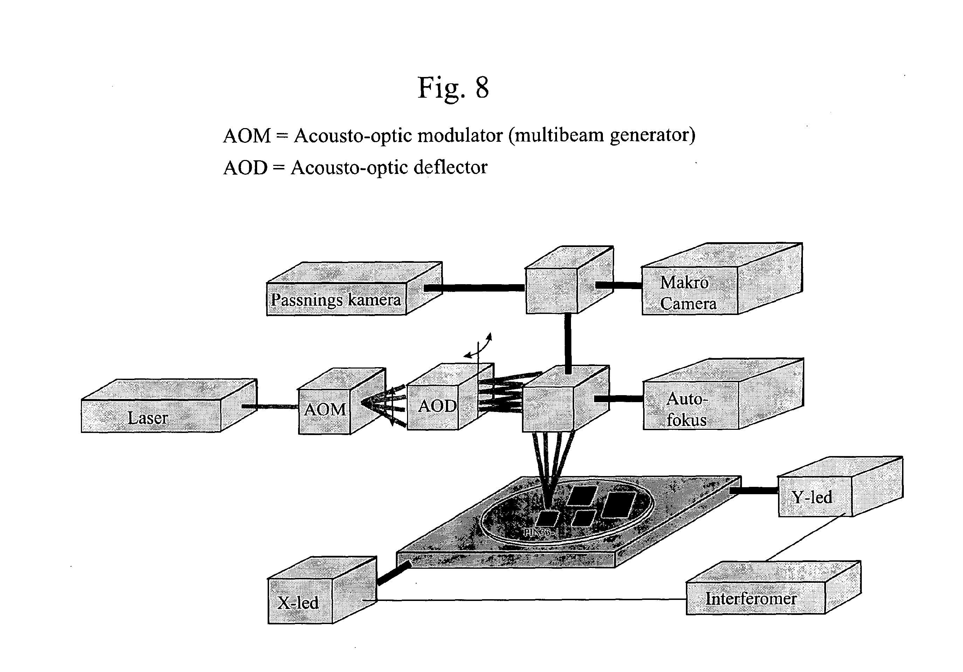

Figure 8 illustrates the principles of an optical DWL equipment, using a laser source. However, other direct write techniques, such as DWL using e-beam lithography, could be used just as well within the scope of the present invention.

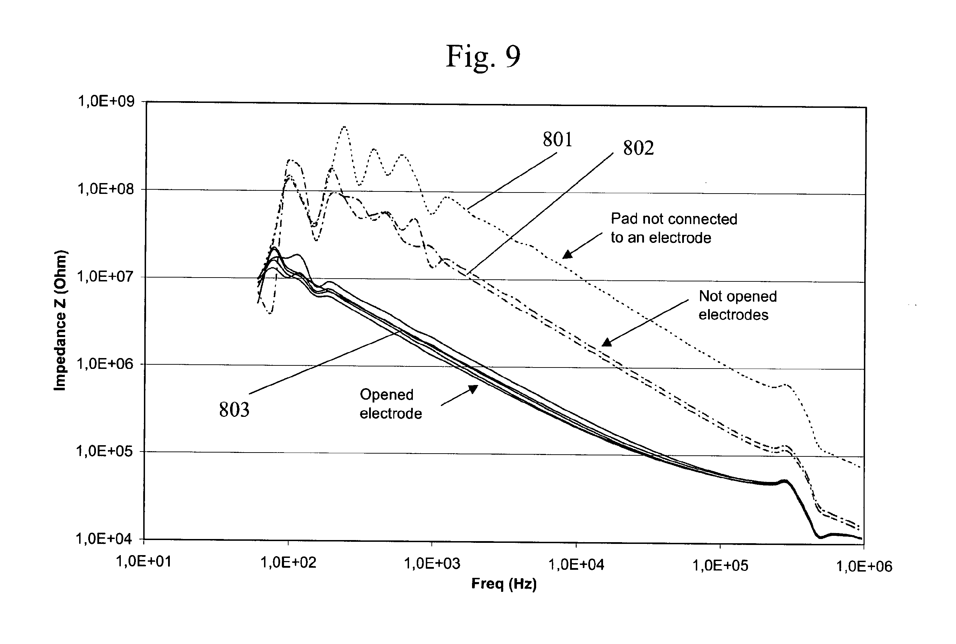

Figure 9 discloses the impedance of an electrode in saline at different frequencies with the electrode window opened and closed, as well as the impedance recorded from a contact pad not connected to an electrode.

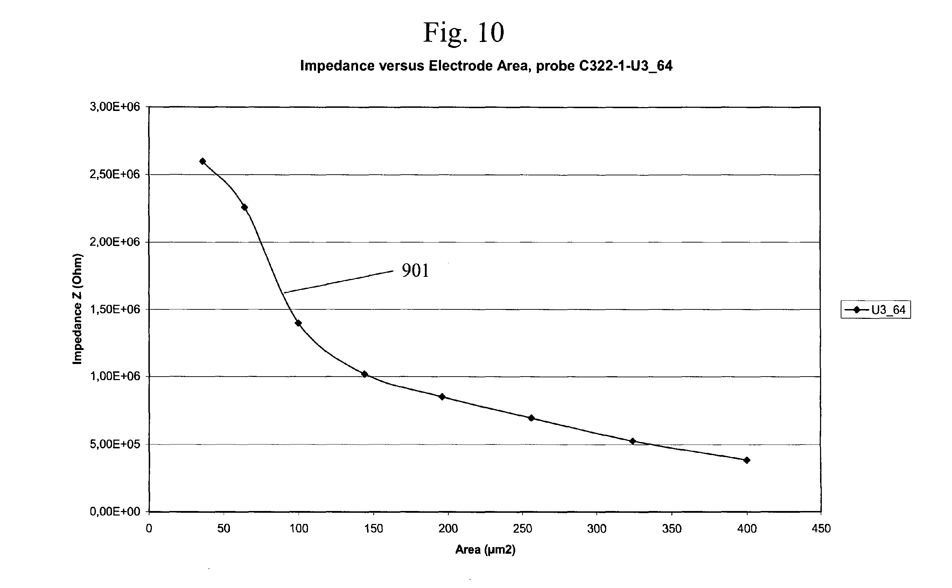

As previously stated, the inventive neural probe provides the possibility to vary the area and thus impedance of the active electrodes. In Figure 10 a diagram is displayed, showing the impedance in saline of an electrode for different electrode window areas measured at 1 kHz signal frequency.

The electrode impedance was characterized in 0.9 % saline by 3 point measurements with an HP 4284A Precision LCR Meter. The results showed approximately 15 times higher impedance for closed compared to opened windows and distinctly decreasing impedance for increasing electrode areas, see Figure a5. If required, the impedance of the closed windows can be further increased by increasing the thickness of the top Si₃N₄-layer.

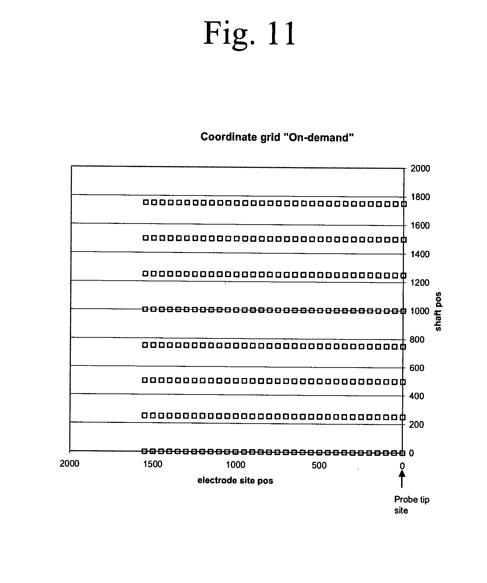

Figure 11 shows a two-dimensional electrode array space that is representative of prior art neural probe designs. On this parameter space a "snap grid", enabled by the invention, has been imposed which covers the space while still keeping the number of grid points within reasonable limits. By using a set of 5 standard designs with 64 electrodes, as disclosed in Figure 13, the parameter space can be almost completely covered, as illustrated in Figure 14.

In addition to the regular electrode patterns shown in the example above, each shaft within the same probe can be programmed to different electrode distributions as well, resulting in an even larger number of optional designs.

Thus, a concept for on-demand manufacture of semi-custom neural probes, possibly using direct write lithography is presented. The concept is demonstrated by the manufacture of microelectrodes with differently spaced and sized electrode windows and evaluated by 3 point impedance measurements.

It is finally assumed that the third design parameter, the shaft length (L), can be varied by inserting longer standard shafts only partially.

In conclusion, a probe is disclosed, the probe comprising at least one shaft and at least one contact pad. Each shaft is suitable for insertion into neural tissue and has a number of electrodes. Each contact pad is in electrical contact with a set of the electrodes and at least one electrode is at least partially covered by an electrically insulating layer. Furthermore a manufacturing process for such a probe is disclosed.

## Claims

1. A method of manufacturing a probe suitable for insertion in neural tissue and comprising at least one shaft, the method comprising the steps of:
providing at least one set of electrodes on the at least one shaft, the electrodes being located at respective electrode sites and the electrodes of each set being electrically interconnected;
applying an electrically insulating layer onto every electrode;
removing at least a part of the electrically insulating layer from every electrode comprised in a desired electrode distribution.

2. A method as claimed in claim 1, in which the desired electrode distribution comprises at most one electrode of each set of electrodes.

3. A method as claimed in claim 1, in which the desired electrode distribution comprises at least one electrode of each set of electrodes.

4. A method as clamed in any of the above claims, in which the desired electrode distribution comprises one electrode from each set of electrodes.

5. A method as clamed in any one of the claims 1-3, in which the desired electrode distribution comprises electrodes from a number of the sets of electrodes less than the total number of sets.

6. A method as claimed in any of the above claims, in which the step of removing the insulating layer involves removing differently sized parts of the insulating layer from different electrodes.

7. A method as claimed in any of the above claims, in which the step of removing at least parts of the electrically insulating layer from the electrodes involves a direct write method.

8. A method as claimed in claim 7, in which the direct write method involves use of a laser beam.

9. A method as claimed in claim 7, in which the direct write method involves use of a particle beam.

10. A method as claimed in any one of the above claims, in which a plurality of shafts are manufactured simultaneously, the shafts being attached to a base plate in a fork-like configuration.

11. A method as claimed in claim 10, in which the step of providing electrodes involves providing equivalent electrode sets to each shaft, the sets of different shafts being electrically insulated from each other.

12. A method as claimed in claim 10 or 11, the desired electrode distribution being equal for each shaft.

13. A method as claimed in claim 10 or 11, the desired electrode distribution being different for different shafts.

14. A method as claimed in any one of the above claims, the method involving simultaneous manufacturing of a number of neural probes.

15. A method as claimed in any one of the above claims, the neural probes being made out one of silicon, metal and plastic.

16. A probe, comprising at least one shaft and at least one contact pad, each shaft being suitable for insertion into neural tissue and having a number of electrodes, and each contact pad being in electrical contact with a set of the electrodes, **characterized in that** at least one electrode is at least partially covered by an electrically insulating layer.

17. A probe as claimed in claim 16, the probe being a neural probe.

18. A probe as claimed in any one of claims 16-17, the probe comprising a number of contact pads.

19. A probe as claimed in any one of claims 16-18, in which at least one of the electrodes if fully covered by an electrically insulating layer.

20. A probe as claimed in any one of claims 16-19, in which every electrode except one of each set of electrodes is fully covered by an electrically insulating layer.

21. A probe as claimed in any one of claims 16-20, the probe comprising at least two shafts, the electrodes on each shafts being electrically insulated from the electrodes on every other shaft.

22. A probe as claimed in any one of claims 16-21, in which the insulating layer on top of the electrodes is removable.

23. A probe as claimed in any one of claims 16-22, the probes being made out any one of silicon, metal and plastic.
